# EUROPEAN PATENT APPLICATION

(11) **EP 1 849 461 A1**
(43) Date of publication of application: **31.10.2007**
(21) Application number: 06290669.8
(22) Date of filing: 21.04.2006
(51) Int. Cl.: A61K 9/48

(54) **Process for manufacturing films**

(71) Applicant: Pfizer Products Inc., Groton, CT 06340 (US)
(72) Inventor: Cade, Dominique, 68000 Colmar (FR); He, Xiongwei, 68280 Andolshelm (FR)
(74) Representative: Dekker, Henrike Cornelie Christine

(57) **Abstract**

The invention relates to a process for preparing a film forming composition, manufacturing films and containers therefrom, in particular for manufacturing hard capsules based on water-soluble cellulose ether derivatives and to hard capsules prepared by said process.

## Description

The present invention relates to a process for preparing a film forming composition, manufacturing films and containers therefrom, in particular for manufacturing hard capsules based on water-soluble cellulose ether derivatives and to hard capsules prepared by said process.

For the industrial manufacture of pharmaceutical capsules gelatine is most preferred for its gelling, film forming and surface active properties. The manufacture of hard gelatine capsules by dip moulding process exploits fully its gelling and film forming abilities. Such capsules are manufactured by dipping mould pins into a hot solution of gelatine, removing the pins from the gelatine solution, allowing the gelatine solution attached on pins to set by cooling, drying and stripping the so-formed shells from the pins. The setting of the solution on the mould pins after dipping is the critical step to obtain a uniform thickness of the capsule shell.

Capsules are widely used in the pharmaceutical industry as well as in the health food supplement market. The main usage thereof is as dosage form for solid, semi-solid, liquid, pellet or herbal preparations. A primary objective of these dosage forms is to have a good disintegration after being administrated in order to enable an effective dissolution of the active substances in the appropriate digestive organ. Consequently, this disintegration characteristic has to remain stable over time when finished products are stored prior to use. The traditional material for forming the capsule shell is gelatin, because it has the correct and quite ideal properties. Nevertheless gelatin has some disadvantages which make it necessary to have other capsule shell materials available.

Cellulose ether derivatives are known as ideal replacement materials for gelatin. It has been tried to use them e.g. on conventional capsule-making machines, but if the cellulose ether derivative is used alone the process needs to be modified because of their temperature related properties. The viscosity of their solutions, when heated from room temperature, first decreases to a minimum value then it increases rapidly to a gel point, which is only a few degrees above the point of minimum viscosity. Various celluloses and manufacturing methods have been used in the past including solvent evaporation to form a film, using thermal gelling process temperatures at temperatures at or above 100°C and then using moulds heated to 200°C using PTFE granules.

The first hydroxyalkyl alkyl cellulose capsules, in particular hydroxypropyl methyl cellulose (HPMC) capsules were made by a thermal gelation process. This process is based on the fact that an aqueous solution of HPMC will gel when heated to a certain temperature. The setting is an indispensable requirement in order to achieve a uniform film thickness distribution. Without the setting the solution will flow down from the pins, no capsule can be produced.

For example EP 0781542 (A2) relates to a method and apparatus for manufacturing pharmaceutical capsules use an aqueous solution of a thermogelling cellulose ether derivative composition and use capsule body pins and capsule cap pins as moulds. The method involves heating the pins, dipping the pins into the solution to cause the solution to gelatinise on the surface of the pins, removing the pins and drying the gelatinised solution on the surface of the pins to form capsule bodies and capsule caps. Pins are heated pre-dip and post-dip to facilitate gelating. Counterflow air is applied to provide drying from the inside and radiant heat is applied from a dome-shelter radiant heater mounted facing the domes of a planar array of pins to ensure the solution is fully gelatinized before drying. It is a disadvantage of this process that the coating on the pins is not gelled or solidified and can fall down from the pins if heating is insufficient or the coating can be wrinkled during gelation if the heating temperature is too high. Furtheron this method requires a special apparatus or operation for heating the moulding pins.

It is evident that with such high pin and drying temperature, the process is difficult to keep stable or consistent. For example, any machine stop will affect seriously the capsule quality.

To overcome the problems and disadvantages associated with thermal gelation methods it has been tried to modify the cellulose ether dipping mixture by using an additional gelling system.

US 5,756,123 relates to a process for manufacturing HPMC capsules wherein the capsule shell being prepared by drying an aqueous solution comprising 18 to 28% by weight of hydroxypropyl-methyl cellulose having a viscosity of 2.4 to 5.4 centistokes as measured in a 2% aqueous solution at 20 °C. as a base, 0.01 to 0.09% by weight of carrageenan as a gelling agent, and 0.05 to 0.6% by weight of at least one ion selected from the group consisting of potassium and calcium ions as a co-gelling agent. The process makes use of conventional dipping technique wherein the immersion solution in which shaping pins are immersed is preferably set at a temperature of 48 °C to 55 °C, especially 51 °C to 53 °C.

Outside this temperature range, the immersion solution would have a finely varying jelly viscosity and thickly or thinly adhere to the pins, failing to form shells of uniform gage. Thereafter the immersion solution adhering to the pins is preferably dried at a temperature of 25 °C to 35 °C for 40 to 60 minutes. Through the drying step, the immersion solution adhering to the pins is concentrated to form hard shells around the pins.

US 5,431,917 relates to a method for producing a hard capsule for pharmaceutical drugs, said method comprising the steps of preparing an aqueous solution of a capsule base containing a water-soluble cellulose derivative in the form of a cellulose ether derivative in which some or all of the hydroxyl groups thereof are substituted with an alkyl group and/or a hydroxyalkyl group, a gelatinizing agent and an auxiliary for gelation, immersing a capsule moulding pin in the aqueous solution of the capsule base, subsequently drawing out the moulding pin in the aqueous solution of the capsule base, subjecting the aqueous solution of the capsule base attached to the outer surface of the moulding pin to gelation at a temperature of 22.5 °C to 25.5 °C, and forming a capsule film on the outer surface of the moulding pin, wherein the solution temperature of the aqueous solution of the capsule base is 48°C. to 55 °C.

At high temperature, the HPMC dipping mixture (dispersion) has very low viscosity as the HPMC substantially does not dissolve. With the cooling, the viscosity increases progressively. From about 55°C, further cooling induces HPMC particles solubilisation, leading a more pronounced increase in the viscosity. At 50°C the increase in the viscosity becomes so sharp that the mixture turns to a jelly-like solution with an extremely high viscosity. This phenomenon could be of the same mechanism to the thermal gelation, the intermolecular hydrophobic interaction between the high substituent portions where 3 hydroxyl groups are all substituted with the methoxyl groups. The viscosity of jelly-like solution is much higher than the workable range of dipping process and prevents the good adhesion of the solution to the capsule-forming pins during dipping and in particular prevents the manufacturing of capsules on the conventional dip moulding process equipment.

In practice, the dipping temperature is kept higher than 50°C in order to avoid the above issues. However, experience revealed that this process is not without weakness and difficulties. When the dipping temperature is close to 50°C, such as <55°C, the viscosity of the dipping mixture is in sharp increase with the temperature cooling. Any change in the dipping mixture temperature will lead to important change in viscosity, and consequently to important change in the capsule dimension, which makes problematic the filling operation on the high speed equipment. On the other side, if the dipping temperature is significantly higher than 50°C, the dipping mixture is a dispersion, most HPMC particles are not dissolved in it. These particles swell or partially dissolve only on the dipping pin after the dipping which reduces the temperature of the HPMC dispersion attached on the pin surface, followed immediately by the formation of gelling system network and capsule drying. All these factors make the solubilisation of HPMC particles very limited; the resulted capsules shell is heterogeneous, with low transparency and poor mechanical performance.

US 6,413,463 relates to method of manufacturing hard capsules which characterized by comprising the steps of dispersing a water-soluble cellulose derivative in hot water and cooling the dispersion to effect dissolution of the water-soluble cellulose derivative in the water, adding and dissolving a gelling agent in the water-soluble cellulose derivative solution to give a capsule- preparing solution, dipping a capsule-forming pin into the capsule-preparing solution at a predetermined temperature, then drawing out the pin and inducing gelation of the capsule-preparing solution adhering to the pin.

However, it is evident that the above process with such cooling and heating back steps is time and energy consuming. Furtheron the long time cooling and heating around 30-40°C is a very favourable condition for bacteria growth.

On the other hand, there is another issue in the recycling of cellulose ether derivative material which has e.g. already been formed into films or capsules or resulting from the capsule cutting for the target length (trims). In fact, the trims or other recycling materials from defective capsules contain already the gelling system. It will gel when the solution is cooled down to room temperature. And the temperature at which the solution is heated up back (35-50°C) is not high enough to dissolve the gel formed at room temperature. Consequently is impossible to recover the gelling ability of the gelling agent contained in the trim.

Since the first patents issued many attempts have been made to manufacture cellulose ether capsules in quantity, using different compositions, with sufficient uniformity to be suitable for filling in modern high-speed filling machines. It has also been tried to manufacture films and other containers of cellulose ether derivatives on conventional machines and/or in industrial scale.

In the following this is explained in more detail on basis of capsule manufacturing as an example. Until today, cellulose ether films, containers or capsules manufactured in quantity suffer imperfections such as haze, wrinkles, starred ends and corrugations, inhomogeneous form, opaqueness. These imperfections result in films or capsules either breaking, failing to separate, providing bad solubilisation or disintegration or jamming in the high-speed filling machine.

Such methods require a strict temperature control of the whole capsule forming process. The setting ability of the film forming mixture, here e.g. the capsule base before immersing the capsule moulding pins, needs to be adjusted so as to obtain uniform capsules. This is disadvantageous both with respect to the equipment and the other resources to be used. To meet these strict temperature control the processes known in the art require a lot of different process steps, e.g. dissolving the cellulose derivative at a certain temperature bringing this mixture to a specific temperature before adding the gelling agent, mixing, addition of further ingredients at specific temperatures and so on. Due to the fact that the entire method is viscosity driven, it is very difficult to find a process window/temperature range where the setting ability of the aqueous capsule base mixture is sufficient, and the ingredients of said mixture are at least partly solubilized. If these conditions are not met, it is impossible to obtain totally homogenous, clear and bubble-free capsule shells. Furthermore, often ageing of the aqueous mixture can become a problem. Aging is a quite general phenomenon which occurs in a wide variety of off-equilibrium materials. Aging affects the properties of materials like mechanical, rheological, magnetic etc. This can for example result in further inhomogeneities.

The present invention has been made under the above-described circumstances, and its object is to provide a process of manufacturing uniform, transparent films useful for hard capsules, containers and in particular capsules made of a water-soluble cellulose derivative such as HPMC without requiring a lot of different process steps, easing the requirements of temperature control and reducing length of time. It is an object of the present invention to provide an economical process for manufacturing uniform transparent films, "hard containers" and in particular hard capsules made of water-soluble cellulose ether derivatives wherein the setting ability of the film forming mixture is sufficient; that is, a process which allows the use of conventional film forming or e.g. dip moulding equipment and gives raise to the production of totally homogenous, transparent and bubble-free containers (capsules) showing a good dissolution profile, disintegration profile and stability. It is a further object of the invention to provide a more simple process useful for industrial scale manufacturing processes and saving time and energy.

To overcome these before mentioned drawbacks and to achieve the above object, the present invention provides a process for manufacturing films or containers thereof, characterized by comprising the steps of dispersing a water-soluble cellulose ether derivative, complete amount of a setting system comprising at least one hydrocolloid and if required one or more gelling aid, in hot water and cooling the dispersion to a temperature of less than 48 °C, preferably this temperature is higher than the setting temperature related to the setting system in the dispersion, so as to obtain a film forming mixture, contacting a surface of a shaping device with the film forming mixture, inducing gelation of the film forming mixture adhering to the surface of a shaping device and subsequently drying the film adhering to the surface of a shaping device.

After drying the film, container or capsule it is removed from the surface of the shaping device, e.g. a mould pin.

In the process of the present invention, first of all a water-soluble cellulose ether derivative and a complete amount of a setting system comprising at least one hydrocolloid and if required a gelling aid, are dispersed in hot water. The temperature of the hot water may be selected as appropriate for the type of water-soluble cellulose ether derivative used, although the temperature is preferably at least 60 °C, more preferably 65 to 85 °C, and most preferably 70 to 85 °C. In another preferred embodiment the temperature of the hot water is 70 to 80 °C. The water-soluble cellulose ether derivative, the complete amount of a setting system and optionally one or more further ingredients, are added in any given order into hot water preferably of a predetermined temperature within the above mentioned temperature range.

Alternatively, the water-soluble cellulose ether derivative, the complete amount of setting system agent and optionally one or more further ingredients, are poured into water having a lower temperature lower than a predetermined temperature, followed by heating to a predetermined temperature within the above mentioned temperature range, i.e. 60°C or above.

Preferably the dispersion is mixed continuously during cooling. The mixing during cooling has two important roles. The first is to minimize the temperature gradient within the mixture.

The second is to avoid the excessively high viscosity observed around temperatures between 40 to 50°C if the mixture is cooled without mixing or without enough mixing. Preferably the mixing is done in an efficient way in order to achieve above tow roles. However, the mixing should be not too strong in order to avoid the generation of bubbles in the dispersion or film forming mixture.

After the water-soluble cellulose ether derivative and the complete amount of a setting system comprising at least one hydrocolloid and if required a gelling aid, in hot water, have been dispersed in hot water, the dispersion is cooled to a temperature of less than 48 °C. Cooling may be carried out by either natural cooling or forced cooling. Hereby, cooling of the hot dispersion to a temperature of less than 48 °C will result in an optimized aqueous capsule-preparing dipping mixture or film-forming mixture which preferably is almost an aqueous capsule-preparing solution.

Preferably the mixture is cooled down to less than 48°C but higher than the setting temperature. The setting temperature is the temperature at which the mixture starts to gel. This can be determined by the sharp increase in the viscosity of the mixture during cooling. A temperature higher than setting temperature avoids the risk of reducing the setting ability by overcooling. This allows in particular the manufacture of hard capsules by conventional dip moulding process. The setting temperature depends on the type of setting system and their relative amounts. Preferably the setting temperature is between 20-45°C.

In this application the terms setting and gelling are used synonymously.

The appropriate cooling time is determined by the effectiveness of cooling without producing a heavy temperature gradient within the mixing vessel and/or causing bubbles in the mixture.

Cooling time preferably is in a range of from 0,5 to 24 hours, more preferably 0,5 to 18 hours, most preferably 1 to 12 hours or 1 to 8 hours. In general, cooling is carried out to a temperature of less than 48 °C, in another embodiment to 47 °C or less and still another embodiment to 45 °C or less.

In an alternative embodiment cooling of the mixture comprising cellulose ether derivative and setting system is carried out down to a temperature of 35 °C or less, preferably 30 °C or less but above the setting temperature within the time limits given above, and thereafter the aqueous film forming mixture is again heated up to a temperature of below 48 °C so as to obtain the aqueous capsule-preparing dipping mixture or film-forming mixture to be used in the subsequent contacting step of a surface of a shaping device with the film forming mixture which is preferably a dipping step.

According to the present invention, cooling of the dispersion can be achieved by adding a quenching agent, e.g. water, to the hot dispersion, or providing an external cooling means or any combination of these measures. In a preferred embodiment of the present invention the external cooling means is a mixer, which can be any mixer well known in the art, e.g. a stirrer.

Thereafter, the film forming mixture is contacted with a surface of a shaping device at a predetermined temperature. The contacting temperature, e.g. temperature of the aqueous dipping mixture, may be selected according to the type of water-soluble cellulose ether derivative and the setting system. Preferred is a temperature from higher than 20 °C up to 48 °C. In a preferred embodiment of the present invention the temperature is within a range of 40 - 47,9 °C and in still another preferred embodiment the contacting (dipping) temperature is within the range of 43-47,8 °C.

In one embodiment of the present invention the shaping device - which is preferably a capsule forming pin - is preconditioned within a specific temperature range. Such a temperature range for example may depend on the combination of the film forming mixture as well as on the desired properties of the final container which is preferably a hard capsule. The temperature of the shaping device should be less than the setting temperature of the film forming mixture. In a preferred embodiment the temperature of the shaping device, before contacting it with the film forming mixture, is preferably less than 40 °C, more preferably within a range of 15 to 35 °C.

In another embodiment of the present invention as the shaping device, preferably a capsule forming pin, a cooled shaping device is used which cooled shaping device preferably has a temperature in the range of from about 10 to 18 °C.

After contacting the shaping device with (dipping device in) the film forming mixture, the shaping device, preferably a capsule forming pin, is drawn out of the aqueous film forming mixture, and the film forming mixture adhering to the pin is subjected to gelation and drying, giving a final film, container or hard capsule.

The gelation temperature depends mainly on the gelling system and its quantity of the film forming mixture. Gelation of the film forming mixture adhering to the shaping device (e.g. pin) can be achieved by cooling on standing. Preferably the drying temperature is 15-40°C at the beginning to ensure a quick setting of the mixture attached to the surface, e.g. a mould pin and in a second step the temperature can be increased to reduce the drying time. However, if the container (capsule) preparing dipping mixture gels under heating, gelation may be effected by heating to a temperature of 50 to 80 °C, for example after the pin has been drawn out.

In one embodiment of the present invention gelation takes place between 20 and 47°C, in a more preferred embodiment it takes place between 30-45 °C or in another one at 34-40°C and in another preferred embodiment between 41-45°C. In another embodiment of the present invention, after drawing out the shaping device, e.g. a pin, an initial drying step at a temperature of usually less than 30 °C and thereafter a second drying step at a temperature usually at a temperature not higher than 40 °C is carried out.

Heating and drying can for example be achieved by using air at a corresponding temperature or using IR radiation or the like.

Preferably the final capsule has a water content of 2 to 8 %.

For the production of hard capsules by dip moulding process, it is important that the film forming (capsule preparing) dipping mixture attached to the mould pins after dipping is prohibited from flowing down the pins. Otherwise the obtained film will not have the desired uniform thickness. The setting of the solution on the mould pins after dipping is the critical step to obtain a uniform thickness of the capsule shell. The addition of a setting system to cellulose ether derivative enables the adaptation of specific and desired gelling properties for a selected process (film forming or dip moulding such as the production of hard HPMC capsules by a conventional dipping process).

The setting system consists of at least one hydrocolloid or mixtures of hydrocolloids and may contain in addition a gelling aid which can be cations and/or sequestering agents.

In a preferred embodiment of the present invention the process is a dip moulding process which is a method to produce flexible products with specific properties at low costs. Dip moulding is very suitable for making complex shapes in various colours and is very interesting for small and medium quantities as well as for great quantities.

In a preferred embodiment of the present invention a process is provided for manufacturing containers, preferably hard capsules, characterized by comprising the steps of dispersing a water-soluble cellulose ether derivative, complete amount of a setting system comprising at least one hydrocolloid and if required one or more gelling aid, in hot water and cooling the dispersion to a temperature of less than 48 °C so as to obtain a film forming mixture, dipping a mould pin into the film forming mixture, inducing gelation of the film forming mixture adhering to the surface of a mould pin and subsequently drying the film adhering to the surface of mould pin.

Non-uniform capsules are until today usually discarded because the current states in the art processes are not suited for recycling these non-uniform capsules. An advantage of the process according to the present invention is that this non-uniform material can be recycled within the process by dispersing it in hot water instead of fresh/new cellulose ether derivative.

In a preferred embodiment of the process according to the present invention, in addition to or instead of fresh cellulose ether derivative as starting material, "recycling" cellulose ether derivative material which has e.g. already been formed into films or capsules can be recycled and re-used in the process claimed according to the present invention (= trims). An advantage of the process according to the present invention is that this the trims can be recycled within the process by dispersing it in hot water instead of fresh/new cellulose ether derivative. As the solution is never cooled down to below the gelling temperature, the total gelling ability of the gelling agent already contained in the recycling materials is totally recovered.

In the process claimed according to the present invention a water-soluble cellulose ether derivative, the complete setting system comprising at least one hydrocolloid and if required a gelling aid, are dispersed in hot water.

Suitable water-soluble cellulose ether derivatives to be used according to the present invention, are alkyl- and/or hydroxyalkyl substituted cellulose ether derivatives with 1 to 4 carbon atoms in the alkyl chains, preferably methyl cellulose, hydroxyethyl cellulose, hydroxypropyl cellulose, hydroxyethylmethyl cellulose, hydroxyethylethyl cellulose, hydroxypropylmethyl cellulose (HPMC) or the like. Especially preferred is HPMC.

The amount of water-soluble cellulose ether derivative to be used according to the present invention, given as the concentration in the aqueous capsule-preparing mixture, is preferably from 8 to 35% by weight, more preferably 15 to 28% by weight based on the weight of the aqueous capsule-preparing mixture. The amount of the water-soluble cellulose ether derivative or mixture of cellulose ether derivatives in the final capsule is preferably 92 to 98% by weight based on the weight of the capsule. The viscosity of the cellulose ether derivative or blend usually is 3 to 15 cps in 2 % aqueous solution at 20°C, preferred 3 to 10, especially preferred 6 cps.

The setting system to be used according to the present invention may be any substance that induces the gelation of the water-soluble cellulose ether derivative used according to the present invention. Suitable examples include a hydrocolloid or mixtures of hydrocolloids.

Suitable hydrocolloids or mixtures producing synergistic properties may be selected from natural seaweeds, natural seed gums, natural plant exudates, natural fruit extracts, biosynthetic gums, gelatines, biosynthetic processed starch or cellulosic materials. Preferred hydrocolloids to be used according to the present invention are polysaccharides.

The preferred polysaccharides according to the present invention are alginates, agar gum, guar gum, locust bean gum (carob), carrageenan, tara gum, gum arabic, ghatti gum, Khaya grandifolia gum, tragacanth gum, karaya gum, pectin, arabian (araban), xanthan, gellan, starch, Konjac mannan, galactomannan, fucoidan, funoran, and other exocellular polysaccharides. Preferred are exocellular polysaccharides.

The preferred exocellular polysaccharides are xanthan, gellan, carrageenan, furcelleran, succinoglycan, scleroglycan, schizophyllan, tamarind gum, curdlan, pectin, locust bean gum (carob), Konjac mannan, gelatin and dextran. Carrageenan can be k-carrageenan, i-carrageenan or λ-carrageenan or mixtures thereof. Preferred are kappa and iota - carrageenan.

In one embodiment of the present invention a more preferred hydrocolloid to be used, is carrageenan, pectin or gellan gum or combinations thereof.

The amount of the hydrocolloid is preferably less than 3%, especially 0.01 to 1% by weight of the aqueous dipping mixture.

Optionally, in addition to water-soluble cellulose ether derivative and hydrocolloid(s), according to the present invention a gelling aid can be added to the mixture to be dispersed in hot water. The gelling aid is selected according to the type of hydrocolloid used. An appropriate combination of hydrocolloid(s) and gelling aid according to the present invention may be called a setting system.

Usually, as gelling aid there may be used a water-soluble compound that provides cations, preferably mono- or divalent cations such as potassium ions, ammonium ions or calcium ions. The cations are preferably selected from K⁺, Na⁺, Li⁺, NH₄⁺, Ca²⁺ or Mg²⁺. illustrative examples thereof include potassium chloride, potassium acetate, ammonium chloride, ammonium acetate and calcium chloride.

The amount of cations is preferably less than 3%, more preferably less than 1 %, and especially preferred less than 0.5% by weight in the aqueous dipping mixture. Suitable ranges are 0.01 to 3 %, and especially preferred 0.01 to 1 % by weight in the aqueous dipping mixture.

Among the setting systems which preferably used according to the present invention, the systems comprising carrageenan with cation or gellan gum with cation are specifically preferred. And the preferred cations are K⁺, Li⁺, Na⁺ or NH₄⁺. Suitable counterions are acetate, sulphate, carbonate or halogen, e.g. Cl⁻, Br⁻, I⁻.

In a preferred embodiment of the present invention the setting system consists of gellan, more preferably together with a gelling aid and in another preferred embodiment it consists of carrageenan and a cation, preferably potassium. While for carrageenan monovalent cations are preferred for gellan are divalent cations are preferred. Preferred divalent cations to be used as gelling aid in the presence of gellan are Ca⁺ and/or Mg⁺. In another preferred embodiment a mixture of gellan and carrageenan is used as a setting system, more preferably including a cation, e.g. K⁺, as a gelling aid. In still another preferred embodiment pectin is used as a setting system. When pectin is used as a setting system divalent ions are preferred as gelling aid - preferably Ca⁺.

Optionally, the film forming mixture contains one or more sequestering agents. The preferred sequestering agents to be used according to the present invention are ethylenediaminetetraacetic acid, acetic acid, boric acid, citric acid, edetic acid, gluconic acid, lactic acid, phosphoric acid, tartaric acid or salts thereof, metaphosphates, dihydroxyethylglycine, lecithin or beta cyclodextrin and combinations thereof. Especially preferred is ethylenediaminetetraacetic acid or salts thereof or citric acid or salts thereof. The sequestering agent may also include a mixture of different sequestering agents. The amount of sequestering agent is preferably less than 3%, especially less than 1 % by weight based on the weight of the aqueous dipping mixture.

Furthermore, one or more additional ingredients may be added to the dispersion and/or during the cooling step and/or before contacting the film forming mixture with the surface of the shaping device. This addition is not limited to a specific stage of the process as long as it is added before contacting a surface of a shaping device with the film forming mixture. These further ingredient(s) can be added all at the same stage or each one at a different stage as well as parts of the same ingredient can be added at different stages.

In a preferred embodiment of the present invention all ingredients are added at the stage when dispersing the cellulose ether derivative and the setting system.

The optionally ingredients may include one or more ingredients selected from the group consisting of colouring agent, surfactant, dissolution enhancer, plasticizer and/or further additives.

In addition, a pharmaceutically or food acceptable colouring agent may be present in the mixture to be dispersed in hot water or may be added at a later stage.

The colouring agents may be selected from azo, quinophthalone, triphenylmethane, xanthene or indigoid dyes, iron oxides or hydroxides, titanium dioxide or natural dyes or mixtures thereof. Examples are patent blue V, acid brilliant green BS, red 2G, azorubine, ponceau 4R, amaranth, D+C red 33, D+C red 22, D+C red 26, D+C red 28, D+C yellow 10, yellow 2 G, FD+C yellow 5, FD+C yellow 6, FD+C red 3, FD+C red 40, FD+C blue 1, FD+C blue 2, FD+C green 3, brilliant black BN, carbon black, iron oxide black, iron oxide red, iron oxide yellow, titanium dioxide, riboflavin, carotenes, anthocyanines, turmeric, cochineal extract, chlorophyllin, canthaxanthin, caramel, or betanin.

If present, the colouring agent may be contained in an amount in the range of from 0 to 5% by weight of the final capsule.

Furthermore, the final film forming mixture may comprise a surfactant. Suitable surfactants to be used according to the present invention may include silicates, silicones, lecithins and mono- and diglycerides and substances which function in a similar manner. The surfactant may be sodium lauryl sulphate (SLS), dioctyl sodium sulfosuccinate (DSS), benzalkonium chloride, benzethonium chloride, cetrimide (trimethyl-tetradecylammonium bromide), fatty acid sugar esters, glyceryl monooleate, polyoxyethylene sorbitan fatty acid esters, polyvinyl alcohol, dimethylpolysiloxan, sorbitan esters or lecithin. Said surfactants preferably are present in the mixture to be dispersed in hot water, in an amount of up to about 0 - 5%, preferably 0-3% by weight, based on the aqueous capsule-preparing dipping mixture. In one embodiment the amount is in the range of 0,1 % and 5% by weight, and more preferably between about 0,1% and 3%, by weight, based on the aqueous capsule-preparing dipping mixture.

Additional ingredients may include a plasticizer. The plasticizer to be used according to the present invention hereby may include an extremely wide variety of water-soluble or water-miscible plasticizers and mixtures of plasticizers, e.g. glycerol, propylene glycol, monoacetin, diacetin, triacetin, glycol diacetate, hydroxypropylglycerol and all analogous polyols and the esters or ether derivatives thereof, together with trimethyl citrate; triethyl citrate, di-n-butyl tartrate and other carboxylic acid derivatives.

In order to obtain an even film, the plasticizer is preferably present in a concentration at which the dispersion does not coagulate. The plasticizer should also have a relatively high boiling point so that there is little plasticizer evaporation and the capsules retain their elasticity and softness.

In one preferred embodiment the plasticizer or mixture of plasticizers used according to the present invention is selected from polyethylene glycol, glycerol, sorbitol, sucrose, corn syrup, fructose, dioctyl-sodium sulfosuccinate, triethyl citrate, tributyl citrate, 1,2-propylenglycol, mono-, di- or triacetates of glycerol, or natural gums. More preferred are glycerol, polyethylene glycol, propylene glycol, citrates and their combinations.

The amount of plasticizer depends on the final application. For hard film formulations, such as for hard capsules, the plasticizer is usually contained in an amount of 0 to 20% in the final capsule.

Plasticisers to be used according to the present invention may also include low molecular weight poly(alkylene oxides), such as, for example, poly(ethylene glycols), poly(propylene glycols), poly(ethylene-propylene glycols), organic plasticizer of low molecular mass, such as, for example, glycerol, pentaerythritol, glycerol monoacetate, diacetate or triacetate, propylene glycol, sorbitol, sodium diethylsulfosuccinate, triethyl citrate and tributyl citrate, Na citrate and other substances which function in a similar manner.

In addition, according to the present invention the final film forming mixture may comprise further or other additives such as extenders, fillers, flavouring agents, stabilizers etc.

In a preferred embodiment of the present invention a process for manufacturing capsules is provided wherein a soluble cellulose ether derivative, preferably HPMC, the complete amount of setting system, which is gellan and optionally a gelling aid, preferably potassium acetate (KAc), are dispersed in hot water. In another preferred embodiment the process for manufacturing capsules comprises the dispersion of a soluble cellulose ether derivative, preferably HPMC, the complete amount of setting system, which is a mixture of gellan and carrageenan and optionally a gelling aid, preferably KAc, in hot water. In another preferred embodiment the process for manufacturing capsules comprises the dispersion of a soluble cellulose ether derivative, preferably HPMC, the complete amount of setting system, which is carrageenan and optionally a gelling aid, preferably KAc, in hot water. Preferably when gellan is used as setting agent, EDTA is added during the above mentioned processes as a sequestering agent. More preferably EDTA is added during the preparation of the dispersion in hot water.

The viscosity of the aqueous capsule preparing dipping mixture is not limited, although it is preferably set within a range of 100 to 10000 mPa.s, and especially 1000 to 8000 mPa.s, at the capsule dipping temperature. In one preferred embodiment the viscosity of mixture mentioned above is in the range of 300 - 5000 mPa.s. This viscosity is the value obtained using a Brookfield-type rotational viscometer.

The process according to the present invention may further comprise a coating step wherein the final film, container or capsule is coated. The coating can be e.g. spray coating. The containers, e.g. capsules, obtained according to the present invention or the final products thereof may be subsequently coated with a suitable coating agent like cellulose acetate phthalate, polyvinyl acetate phthalate, methacrylic acid polymers, hypromellose phthalate, hydroxypropylmethyl cellulose phthalate, hydroxyalkyl methyl cellulose phthalate or mixtures thereof to provide e.g. enteric properties.

The containers, e.g. capsules, obtained according to the present invention may be used for the production of containers for providing unit dosage forms for example for agrochemicals, seeds, herbs, foodstuffs, dyestuffs, pharmaceuticals, flavouring agents and the like.

The film forming mixture manufactured by a process of the present invention comprising the steps of dispersing a water-soluble cellulose ether derivative and the complete amount of a setting system comprising at least one gelling agent and if required one or more gelling aid, in hot water and cooling the dispersion to a temperature of less than 48 °C so as to obtain a film forming mixture, can e.g. be used for casting flat films or spray coating or as a sealing liquid for e.g. capsule halves.

The improved properties of the films, containers or capsules, like good transparency, good dissolution profile, good disintegration profile, good mechanical performance, etc, prepared according to the present invention are demonstrated by the following examples.

### Examples

The following examples are not meant to be exhaustive or limiting. They are intended to illustrate the present invention.

HPMC used in the examples has a viscosity of 6 mPas of an 2% aqueous solution at 20°C.

### Example 1 (HPMC dispersion without setting system)

This example illustrates the role of mixing during cooling of the hot HPMC dispersion in order to avoid forming of a jelly-like solution and the excessive viscosity building.

The following graph illustrates the viscosity evolution with the time after a 17% HPMC dispersion is quenched to 47°C. The viscosity is measured by Brookfield type viscometer.

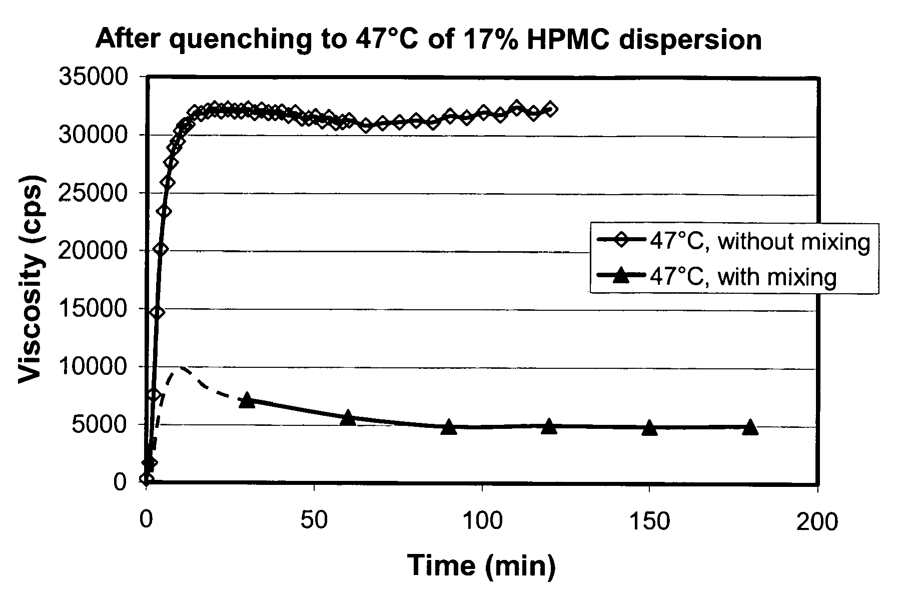

Above results illustrate clearly that without mixing, a jelly-like solution is obtained with an excessively high viscosity which is far beyond the workable viscosity range for dipping process; whereas the viscosity remains at quite acceptable range when with mixing.

### Example 2 (HPMC with setting system)

In a reactor, 4.15 kg of purified water is heated to above 70°C, then 2 grams (0.04% in the final dipping solution) of EDTA disodium, 7 grams (0.14%)of potassium acetate and 3.5 grams (0.07%) of gellan gum are dissolved in the water under mixing, followed by the dispersing of 850 grams (17%) of HPMC powder. After the total dispersing of HPMC powder, reduce the mixing to allow the debubbling of the obtained dispersion. Finally the dispersion is cooled down to 47°C under constant mixing. A quite strong mixing is preferred during the cooling as far as it does not create bubbles.

The graph illustrates that the obtained dipping solution of the present example has a setting (gelling) temperature of 43°C, its viscosity is within the workable viscosity range at temperature between 43-48°C which are the possible dipping temperature window of the current formula.

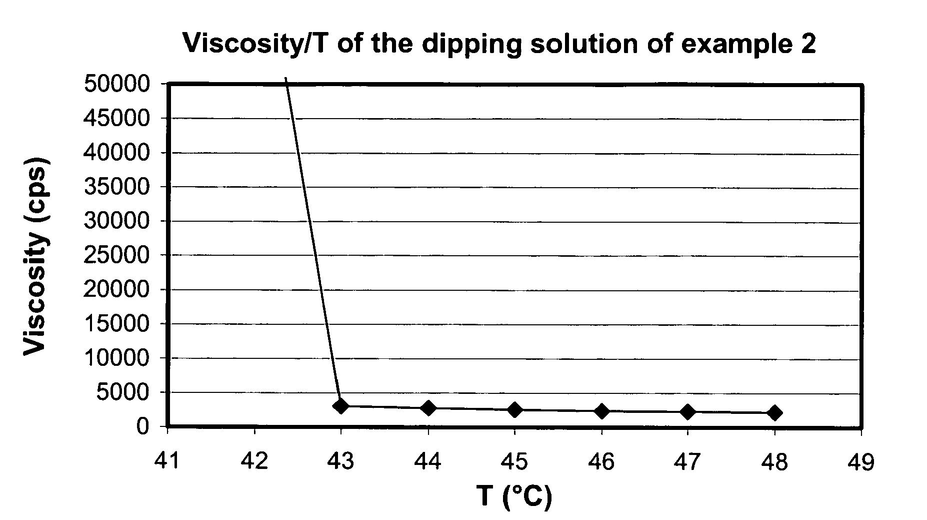

The obtained dipping solution is filled into a dipping dish of a hard gelatin capsule manufacturing pilot equipment, and high quality of hard HPMC capsules are produced with similar process conditions to hard gelatin capsule manufacturing while keeping the dipping solution in the dipping dish at 47°C.

### Example 3 (HPMC with setting system with quenching step)

In a reactor, 3.0 kg of purified water is heated to above 70°C, then 2 grams of EDTA disodium, 7 grams of potassium acetate and 3.5 grams of gellan gum are dissolved in the water under mixing, followed by the dispersing of 850 grams of HPMC powder and the debubbling of the obtained dispersion. The dispersion is then cooled down to 47°C under constant mixing. At the beginning of the cooling, 1.15 kg of purified water at room temperature is added into the reactor in order to accelerate the cooling.

The obtained dipping solution has the same behaviour than the one of example 2. Hard HPMC capsules of the same quality than those in the example 2 are produced under the same conditions than the example 2.

### Example 4 (HPMC with setting system with using recycled material)

In a reactor, 4.15 kg of purified water is heated to above 70°C, then 1 gram of EDTA disodium, 3.5 grams of potassium acetate and 1.75 gram of gellan gum are dissolved in the water under mixing, followed by the dispersing of 425 grams HPMC powder and 430 grams of hard HPMC capsule trims from the example 2. The obtained dispersion is then mixed with a high shearing mixer (Ultra-Turax) during 5 minutes to reduce the trim particle size and to facilitate the dissolving of the gellan gum already present in the recycling trim. After debubbling, the dispersion is cooled down to 47°C as in the example 2.

The obtained dipping mixture has the same composition and behaviour than the one of example 2. Hard HPMC capsules of the same quality than those in the example 2 are produced under the same conditions than the example 2.

### Capsule performances

### • Transparency

Following results illustrated the high transparency of the hard HPMC capsules issued of the current invention.

| **Capsule** | **Transparency** |
|---|---|
| Example 2 | 80% |
| Example 4, with 50% trim recycling | 79% |
| Hard gelatin capsule | 81% |

The transparency is measured by photo spectrometer at 650 nm on the bodies of size 0 capsule.

### • Dissolution

Dissolution test results of capsules filled with acetaminophen in deionised water at 37°C (USP XXIII dissolution) are represented in the following graph.

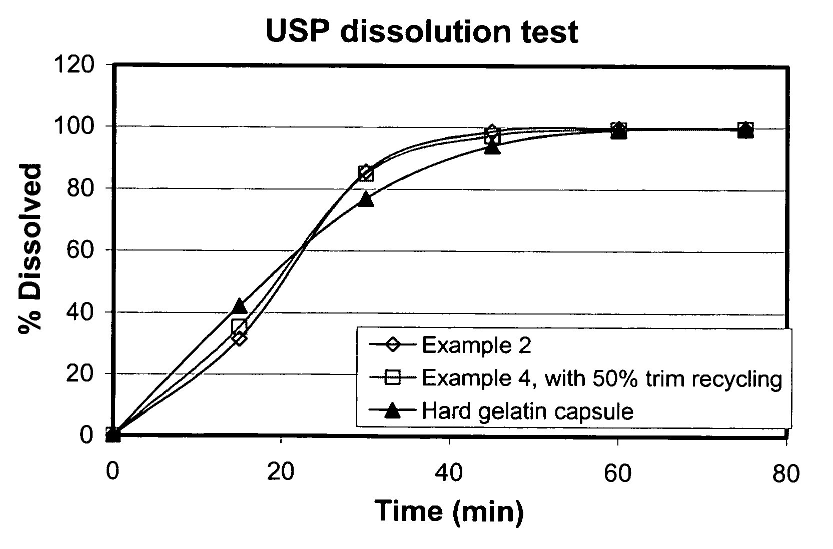

### • Disintegration

The disintegration behaviour has been compared with conventional HPMC capsules at a pH of 1.2 at 37 °C.

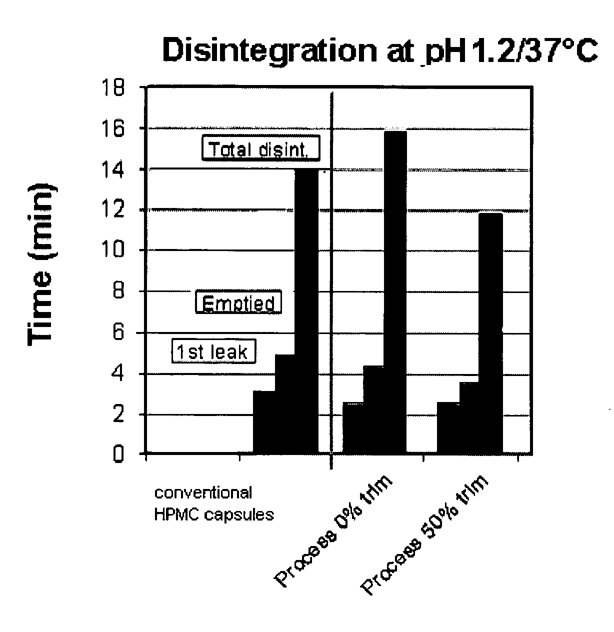

### • Mechanical performance

Capsule mechanical performance is evaluated by an impact test. This test consists of impacting empty capsules in pre-locked position one by one with a weight of 100 g dropping from a height of 80 mm. 50 capsules were used for each sample, and from the number of broken capsules, the % broken is determined. Before the impact test, the capsules were previously equilibrated at different relative humidity at 22°C.

The results confirmed that the capsules from examples 2 & 4 are not brittle at all even at extremely low relative humidity conditions.

| | **Impact test** | | | | | |
|---|---|---|---|---|---|---|
| Equilibrium relative humidity at 22°C | **Example 2** | | **Example 4** | | **Standard hard gelatin capsule** | |
| | % Broken | Water content (%) | % Broken | Water content (%) | % Broken | Water content (%) |
| 2.5 % RH | **0** | 1.0 | **0** | 0.8 | **20** | 8.5 |
| 10 % RH | **0** | 1.5 | **0** | 1.7 | **10** | 10.2 |
| 23 % RH | **0** | 3.0 | **0** | 3.0 | **6** | 11.4 |
| 33 % RH | **0** | 4.0 | **0** | 4.0 | **2** | 12.3 |
| 45 % RH | **0** | 5.4 | **0** | 5.6 | **2** | 12.9 |

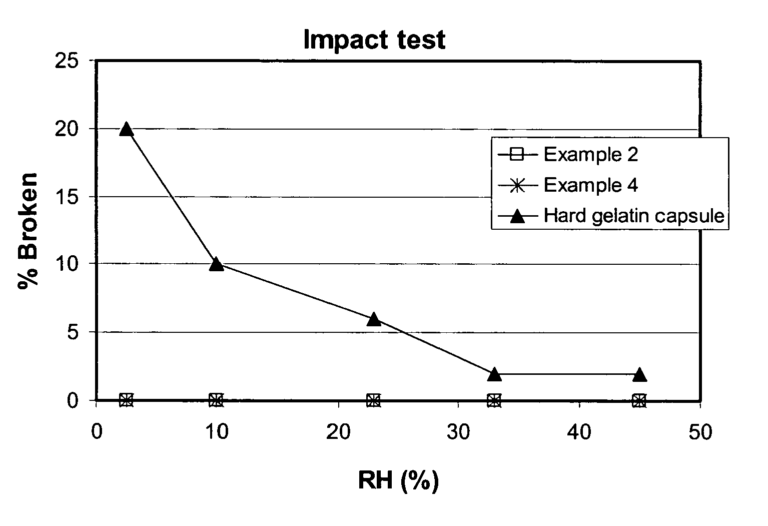

## Claims

1. A process for manufacturing films useful for hard capsules or hard containers thereof, **characterized by** comprising the steps of
A process for manufacturing films, containers, in particular hard capsules thereof, **characterized by** comprising the steps of
• dispersing a) a water-soluble cellulose ether derivative and b) complete amount of a setting system comprising at least one hydrocolloid or a mixture of hydrocolloids and if required one or more gelling aid, in hot water and
• mixing and cooling the dispersion to a temperature of less than 48 °C so as to obtain a film forming mixture,
• contacting a surface of a shaping device with the film forming mixture,
• inducing gelation of the film forming mixture adhering to the surface of a shaping device and
• subsequently drying the film adhering to the surface of a shaping device.

2. A process of claim 1, wherein the cellulose ether derivative is selected from alkyl- and/or hydroxyalkyl substituted cellulose ethers with 1 to 4 carbon atoms in the alkyl chains.

3. A process of any one of claims 1 or 2, wherein the cellulose ether derivative is selected from methyl cellulose, hydroxyethyl cellulose, hydroxypropyl cellulose, hydroxyethylmethyl cellulose, hydroxyethylethyl cellulose, hydroxypropylmethyl cellulose (HPMC).

4. A process of any of the preceding claims, wherein the cellulose ether derivative is hydroxypropyl methyl cellulose.

5. A process of any of the preceding claims, wherein the hydrocolloid or a mixture of hydrocolloids is selected from natural seaweeds, natural seed gums, natural plant exudates, natural fruit extracts, biosynthetic gums, gelatines, biosynthetic processed starch or cellulosic materials, preferred are polysaccharides.

6. A process of any of the preceding claims, wherein the hydrocolloid is a polysaccharide or mixtures thereof selected from a group consisting of alginates, agar gum, guar gum, locust bean gum (carob), carrageenan, tara gum, gum arabic, ghatti gum, Khaya grandifolia gum, tragacanth gum, karaya gum, pectin, arabian (araban), xanthan, gellan, starch, Konjac mannan, galactomannan, fucoidan, funoran, and other exocellular polysaccharides.

7. A process of any of the preceding claims, wherein the hydrocolloid is a polysaccharide selected from xanthan, gellan, furcelleran, succinoglycan, scleroglycan, schizophyllan, tamarind gum, curdlan, carrageenan, pectin, locust bean gum (carob), Konjac mannan, gelatin and dextran

8. A process of any of the preceding claims wherein the gelling aid is a cation or mixtures of cations.

9. A process of any of the preceding claims wherein the gelling aid is a mono- or divalent cation.

10. A process of any of the preceding claims wherein the gelling aid is a cation selected from the group consisting of K⁺, Na⁺, Li⁺, NH₄⁺, Ca⁺⁺ or Mg⁺⁺.

11. A process of any of the preceding claims further comprising a sequestering agent or mixture of sequestering agents.

12. A process of of claim 11 wherein the sequestering agent or mixtures thereof is selected from a group consisting of ethylenediaminetetraacetic acid, acetic acid, boric acid, citric acid, edetic acid, gluconic acid, lactic acid, phosphoric acid, tartaric acid or salts thereof, methaphosphates, dihydroxyethylglycine, lecithin or beta cyclodextrin and combinations thereof.

13. A process of any of the preceding claim, wherein the shaping device is a dip moulding form (tool) which is dipped into the film forming mixture.

14. A process of any of the preceding claim, wherein the shaping device is a mould pin which is dipped into the film forming mixture.

15. The process of any of the preceding claims, wherein the container to be formed is a hard capsule.

16. A process of any of the preceding claims, wherein one or more additional ingredients are added to the hot dispersion and/or during the cooling step and/or before contacting the film forming mixture with the surface of the shaping device.

17. A process of claims 16 wherein additional ingredient(s) is/are added to the hot dispersion.

18. A process of claims 16 or 17, wherein additional ingredient(s) is/are added during cooling.

19. A process of claims 16 to 18, wherein additional ingredient(s) is/are added before contacting step.

20. A process of any of claims 16 to 19, wherein the additional ingredients are selected from the group consisting of plasticizer, surfactant, colouring agent or mixtures thereof.

21. A process of any of claims 16 to 20, wherein an additional ingredient is a colouring agent or a mixture of colouring agents selected from a group consisting of azo-, quinophthalone-, triphenylmethane-, xanthene- or indigoid dyes, iron oxides or hydroxides, titanium dioxide or natural dyes.

22. A process of any of claims 16 to 21, wherein an additional ingredient is a colouring agent or a mixture of colouring agents selected from a group consisting of patent blue V, acid brilliant green BS, red 2G, azorubine, ponceau 4R, amaranth, D+C red 33, D+C red 22, D+C red 26, D+C red 28, D+C yellow 10, yellow 2 G, FD+C yellow 5, FD+C yellow 6, FD+C red 3, FD+C red 40, FD+C blue 1, FD+C blue 2, FD+C green 3, or brilliant black BN.

23. A process of any of claims 16 to 22, wherein an additional ingredient is a colouring agent or a mixture of colouring agents selected from a group consisting of carbon black, iron oxide black, iron oxide red, iron oxide yellow, titanium dioxide, riboflavin, carotenes, anthocyanines, turmeric, cochineal extract, clorophyllin, canthaxanthin, caramel, or betanin.

24. A process of any of claims 16 to 23, wherein an additional ingredient is one or more plasticizer selected from the group consisting of polyethylene glycol, glycerol, sorbitol, sucrose, corn syrup, fructose, dioctyl-sodium sulfosuccinate, triethyl citrate, tributyl citrate, 1,2-propylenglycol, mono-, di- or triacetates of glycerol, or natural gums, poly(alkylene oxides), such as, for example, poly(ethylene glycols), poly(propylene glycols), poly(ethylene-propylene glycols), organic plasticiser of low molecular mass, such as, for example, glycerol, pentaerythritol, glycerol monoacetate, diacetate or triacetate, propylene glycol, Sorbitol, sodium diethylsulfosuccinate, triethyl citrate and tributyl citrate.

25. A process of any of claims 16 to 24, wherein an additional ingredient is a surfactant selected from the group consisting of sodium lauryl sulphate (SLS), dioctyl sodium sulfosuccinate (DSS), benzalkonium chloride, benzethonium chloride, cetrimide (trimethyl-tetradecylammonium bromide), fatty acid sugar esters, glyceryl monooleate, polyoxyethylene sorbitan fatty acid esters, polyvinyl alcohol, dimethylpolysiloxan, sorbitan esters, lecithin or mixtures thereof.

26. A process of any of the preceding claims wherein the hot water has a temperature of 60°C or above.

27. A process of any of the preceding claims wherein the hot water has a temperature of 65 to 85 °C.

28. A process of any of the preceding claims wherein the dispersion is cooled to a temperature below 48°C but higher than the setting temperature of the setting system.

29. A process of any of the preceding claims wherein the dispersion is cooled to a temperature below 48°C within 0,5 - 24 hours.

30. A process of any of the preceding claims wherein the dispersion is cooled to a temperature below 48°C within 1 - 8 hours.

31. A process of any of the preceding claims wherein the gelation (setting) takes place at a temperature between 20-47°C.

32. A process of any of claims 1 to 31 wherein the gelation takes place at a temperature between 30 - 45 °C.

33. A process of any of the preceding claims wherein the dispersion is cooled to a temperature below 40 °C and than reheated up to temperature below 48°C.

34. A process of any of the preceding claims wherein the dipping temperature of the mixture is 25-47.9 °C.

35. A process of any of the preceding claims wherein drying - at least at the initial drying step - takes place at a temperature between 15-40 °C.

36. Hard capsules obtained by a process as claimed in any one of the preceding claims.
